# EUROPÄISCHE PATENTSCHRIFT

(11) **EP 3 227 265 B1**
(45) Veröffentlichungstag und Bekanntmachung des Hinweises auf die Patenterteilung: **17.10.2018**
(21) Anmeldenummer: 15804377.8
(22) Anmeldetag: 30.11.2015
(51) Int. Cl.: C07D 263/38

(54) **VERFAHREN ZUR HERSTELLUNG VON HEONON(METH)ACRYLAT**
METHOD FOR PRODUCING HEONON(METH)ACRYLATE
PROCÉDÉ DE PRODUCTION DE (MÉTH)ACRYLATE D'HÉONON

(30) Priorität: 01.12.2014 US 201462085660 P
(43) Veröffentlichungstag der Anmeldung: 11.10.2017
(73) Patentinhaber: BASF SE, 67056 Ludwigshafen (DE)
(72) Erfinder: MISSKE, Andrea, 67346 Speyer (DE); FLEISCHHAKER, Friederike, 67065 Ludwigshafen (DE); FLECKENSTEIN, Christoph, 63579 Freigericht (DE); KALLER, Martin, 68163 Mannheim (DE); STENGEL, Ulrik, 69488 Birkenau (DE); BLANCHOT, Mathieu, 67245 Lambsheim (DE); NAIR, Ritesh, 69115 Heidelberg (DE)
(86) Internationale Anmeldenummer: PCT/EP2015/078090
(87) Internationale Veröffentlichungsnummer: WO 2016/087384

(56) Entgegenhaltungen:
- WO-A2-2009/080380
- JP-A- 2010 248 310
- ZHEN WENYUAN ET AL: "Preparation and properties of functional UV curable monomers", TULIAO GONGYE - PAINT AND COATINGS INDUSTRY, HUAGONGBU TULIAO GONGYE YANJIUSUO, NANJING, CN, Bd. 39, Nr. 8, 1. Januar 2009 (2009-01-01) , Seiten 15-20, XP009187981, ISSN: 0253-4312

## Beschreibung

Die Erfindung betrifft ein Verfahren zur Herstellung von Heonon(meth)acrylat durch Umesterung von Alkyl(meth)acrylat mit Heonon.

Polymere oder Copolymere, die auf Basis von verzweigten oder linearen C₈-C₂₄-(Meth)acrylaten hergestellt sind, sind in Form von Polymerdispersionen von erheblicher wirtschaftlicher Bedeutung. Das (Meth)acrylat von 2-Hydroxyethyloxazolidinon (Heonon(meth)acrylat) wird zur Nachvernetzung von Polymeren eingesetzt. Sie finden beispielsweise Anwendung als Klebstoffe, Schmiermittel, Ölfeldchemikalien, Anstrichmittel, Textil-, Leder- oder Papierhilfsmittel. Methacrylsäure und (Meth)acrylat sind Sammelbezeichnungen für Acrylsäure und Methacrylsäure bzw. für Acrylat und Methacrylat.

Höhere Alkyl(meth)acrylate können durch katalytische Umesterung von Methyl(meth)acrylat mit den entsprechenden langkettigen Alkanolen erhalten werden. Dabei wird in Gegenwart eines Stabilisators (Polymerisationsinhibitors) gearbeitet.

DE 2 317 226 A1 offenbart ein Verfahren zur Herstellung von (Meth)acrylsäureestern aus einem Gemisch von C₁₀-C₁₈-Alkanolen durch Umesterung von Methyl(meth)acrylat in Gegenwart von Titanalkoholat als Katalysator und 2,6-Di-tert.-butylparakresol (TBK) als Stabilisator. Dabei wird in Gegenwart von Aktivkohle gearbeitet. Nach Beendigung der Reaktion wird Wasser zugegeben, wodurch das Titanalkoholat zu Titanhydroxid/-oxid hydrolysiert wird, welches an die Aktivkohle adsorbiert. Der Feststoff wird abfiltriert und das Reaktionsprodukt einer Wasserdampfdestillation unterzogen.

WO 2009/080380 offenbart ein Verfahren zur Herstellung von Methacrylaten von C₆-C₂₂-Alkoholen durch Umesterung von Methyl(meth)acrylat mit den entsprechenden Alkoholen in Gegenwart von Titanalkoholat als Katalysator. In Beispiel 1 wird Methylmethacrylat mit 2-Ethylhexanol in Gegenwart von Hydrochinonmonomethylether (MEHQ) als Stabilisator und Tetraisopropyltitanat als Katalysator umgesetzt. Dabei wird ein azeotropes Gemisch aus Methanol/Methylmethacrylat abdestilliert. Nach Abdestillieren von nicht umgesetztem Methylmethacrylat wird das Katalysator enthaltende 2-Ethylhexylmethacrylat einer Reindestillation im Vakuum (ca. 30 mbar) unterworfen. Dabei wird 2-Ethylhexylmethacrylat mit einer Reinheit von 99,4 % erhalten.

Bei der Veresterung von (Meth)acrylsäure oder Umesterung von (Meth)acrylsäureestern mit langkettigen Alkanolen können durch Michael-Addition in nicht unerheblichem Maße Nebenprodukte gebildet werden. Nebenprodukte sind Di- oder Oligo(meth)acrylsäurealkylester oder Oxiester der (Meth)acrylsäureester sowohl des Edukt- als auch des Produktesters. Diese stellen in Bezug auf das Zielprodukt Hochsieder dar. Alkyl(meth)acrylate langkettiger Alkanole lassen sich von diesen Nebenprodukten nur durch Vakuumdestillation abtrennen, wobei ab einer bestimmten Kohlenstoffzahl der umgesetzten Alkanole eine Abtrennung nur noch im Hochvakuum und damit wirtschaftlich überhaupt nicht mehr möglich ist. Weiterhin müssen auch der eingesetzte Katalysator und der Stabilisator von dem Produkt abgetrennt werden. Sofern der Siedepunkt des Zielproduktes nicht zu hoch ist, wird im Allgemeinen eine abschließende Reindestillation des Zielproduktes durchgeführt.

Heonon(meth)acrylat (2-Hydroxyethyloxazolidinon(meth)acrylat) wird zur Nachvernetzung von Polymeren, beispielsweise von Polyacrylaten für Superabsorber, eingesetzt. Besondere Bedeutung hat das Heononacrylat. Heononacrylat hat die nachstehende Strukturformel:

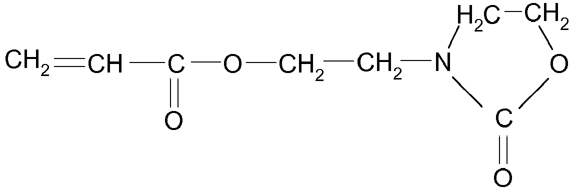

Bei der Herstellung von Heonon(meth)acrylat durch Umesterung von Alkyl(meth)acrylat mit Heonon (2-Hydroxyethyloxazolidinon) stellt sich das Problem der Bildung von Michael-Addukten als Nebenprodukte der Umesterungsreaktion in besonderem Maße.

Aufgabe der Erfindung ist es, ein Verfahren zur Herstellung von Heonon(meth)acrylat durch Umesterung von Alkyl(meth)acrylat mit Heonon bereitzustellen, bei dem nur in geringem Umfang Nebenprodukte gebildet werden.

Gelöst wird die Aufgabe durch ein Verfahren zur Herstellung von Heonon(meth)acrylat durch Umesterung von Alkyl(meth)acrylat mit Heonon, umfassend die Schritte:
(i) Reagieren lassen von Alkyl(meth)acrylat mit Heonon in Gegenwart eines Titan(IV) oder Zirkonium(IV) enthaltenden Katalysators und eines Stabilisators in Gegenwart eines Schleppmittels, das mit dem in dem Alkyl(meth)acrylat gebundenen Alkohol ein Azeotrop bildet,
(ii) kontinuierliches Abdestillieren des Azeotrops aus Schleppmittel und Alkohol, wobei die Schritte (i) und (ii) gleichzeitig durchgeführt werden, bis Heonon im Wesentlichen vollständig umgesetzt ist,
(iii) Zugabe von Wasser zu dem in den Schritten (i) und (ii) erhaltenen, Heonon(meth)acrylat enthaltenden Produktgemisch und Abtrennung von Hydrolysaten des Titan(IV) oder Zirkonium(IV) enthaltende Katalysators,
(iv) Abdestillieren von nicht umgesetztem Alkyl(meth)acrylat und Schleppmittel aus dem Produktgemisch,
(v) Abdestillieren von Wasser aus dem Produktgemisch,
wobei Schritt (iv) auch vor Schritt (iii) durchgeführt werden kann und die Schritte (iv) und (v) auch in einem Destillationsschritt durchgeführt werden können,
dadurch gekennzeichnet, dass die Schritte (i) und (ii) in Gegenwart einer zusätzlichen anorganischen oder organischen Säure durchgeführt werden.

Überraschenderweise wurde gefunden, dass durch Umesterung von Alkyl(meth)acrylat mit Heonon in Gegenwart eines Titan(IV) oder Zirkonium(IV) enthaltenden Katalysators ganz überwiegend das Heonon(meth)acrylat gebildet wird, wenn in Gegenwart einer anorganischen oder organischen Säure gearbeitet wird. Michael-Addukte werden nicht in nennenswertem Ausmaß gebildet.

Im Allgemeinen entstehen Michael-Addukte an Alkyl(meth)acrylat in Mengen von < 0,5 Gew.-%, bevorzugt < 0,1 Gew.-%, bezogen auf das gebildete Heonon(meth)acrylat. Im Allgemeinen wird nach Schritt (v) ein Heonon(meth)acrylat mit einem Nebenproduktgehalt von maximal 2 Gew.-% erhalten.

Unter Michael-Addukten werden die 1,4-Additionsprodukte des Alkohols Heonon an das Eduktmonomer Alkyl(meth)acrylat oder Zielmonomer Heonon(meth)acrylat verstanden. Diese werden auch als Oxiester bezeichnet. Unter Nebenprodukten werden neben den Michael-Addukten an Alkyl(meth)acrylat weitere Verbindungen verstanden, die nicht das Zielprodukt Heonon(meth)acrylat darstellen. Der Gehalt an Nebenprodukten an dem nach Schritt (v) erhaltenen Produkt beträgt vorzugsweise < 2 Gew.-%. Daneben kann das nach Schritt (v) erhaltene Produkt nicht umgesetztes Heonon enthalten. Dieses stellt kein Nebenprodukt dar. Im Allgemeinen beträgt der Gehalt des nach Schritt (v) erhaltenen Produktes an Heonon bis zu 3 Gew.-%, bevorzugt bis zu 2 Gew.-%. Daneben kann das nach Schritt (v) erhaltene Produkt noch Spuren von Schleppmittel, Alkyl(meth)acrylat und Wasser enthalten. Diese stellen ebenfalls keine Nebenprodukte dar und können in Mengen von insgesamt bis zu 2 Gew.-%, bevorzugt bis zu 1 Gew.-% in dem nach Schritt (v) erhaltenen Produkt enthalten sein.

Die Menge aller Nebenkomponenten (einschließlich Nebenprodukten, Heonon, Schleppmittel, Alkyl(meth)acrylat, Wasser) an dem nach Schritt (v) erhaltenen Produkt beträgt im Allgemeinen bis zu 6 Gew.-%, bevorzugt bis zu 4 Gew.-%.

Geeignete Alkyl(meth)acrylate sind die C₁-C₄-Alkyl(meth)acrylate. Im Allgemeinen werden das Methyl(meth)acrylat oder Ethyl(meth)acrylat eingesetzt, wobei bei der Umesterungsreaktion Methanol oder Ethanol als Alkohole freigesetzt werden.

Die Reaktion von Alkyl(meth)acrylat mit Heonon erfolgt in Gegenwart eines Titan(IV) oder Zirkonium(IV) enthaltenden Katalysators. Geeignete Titan(IV) oder Zirkonium(IV) enthaltende Katalysatoren sind die Ti(IV)- bzw. Zr(IV)-tetraalkoxylate von linearen oder verzweigten C₁-C₆-Alkoholen, vorzugsweise Tetraisopropylate, Tetrabutylate sowie das Metallat des eingesetzten Eduktalkohols oder Mischungen hieraus. Auch mit unterschiedlichen Alkoholen oder mit Acetylacetonat substituierte Metallate sind möglich.

Die Schritte (i) und (ii) werden weiterhin in Gegenwart einer anorganischen oder organischen Säure durchgeführt. Besonders geeignete anorganische Säuren sind Phosphorsäure, Schwefelsäure, Salpetersäure, Salzsäure, ganz besonders geeignet sind Phosphorsäure und Schwefelsäure. Besonders geeignete organische Säuren sind Acrylsäure, Methacrylsäure und Essigsäure. Die Säure wird im Allgemeinen in Mengen von 0,01 bis 5 Gew.-%, bevorzugt 0,1 bis 1 Gew.-%, bezogen auf die Gesamtmenge der im Reaktionsgemisch enthaltenen Komponenten, eingesetzt.

Die Reaktion von Alkyl(meth)acrylat mit Heonon erfolgt weiterhin in Gegenwart eines oder mehrerer Stabilisatoren (Polymerisationsinhibitoren). Geeignete Stabilisatoren können beispielsweise N-Oxide (Nitroxyl- oder N-Oxyl-Radikale, also Verbindungen, die wenigstens eine > N-O-Gruppe aufweisen), wie z. B. 4-Hydroxy-2,2,6,6-tetramethylpiperidin-N-oxyl, 4-Oxo-2,2,6,6-tetramethylpiperidin-N-oxyl, 4-Acetoxy-2,2,6,6-tetramethyl-piperidin-N-oxyl, 2,2,6,6-Tetramethylpiperidin-N-oxyl, Bis(1-oxyl-2,2,6,6-tetramethylpiperidine-4-yl)sebacat, 4,4',4"-Tris(2,2,6,6-tetramethyl-piperidin-N-oxyl)-phosphit oder 3-Oxo-2,2,5,5-tetramethylpyrrolidin-N-oxyl; ein- oder mehrwertige Phenole, die ggf. eine oder mehrere Alkylgruppen aufweisen, wie z.B. Alkylphenole, beispielsweise o-, m- oder p-Kresol (Methylphenol), 2-tert.-Butylphenol, 4-tert.-Butylphenol, 2,4-Di-tert.-butylphenol, 2-Methyl-4-tert.-butylphenol, 2-tert.-Butyl-4-methylphenol, 2,6-tert.-Butyl-4-methylphenol, 4-tert.-Butyl-2,6-dimethylphenol oder 6-tert.-Butyl-2,4-dimethylphenol; Chinone, wie z.B. Hydrochinon, Hydrochinonmonomethylether, 2-Methylhydrochinon oder 2,5-Di-tert.-Butylhydrochinon; Hydroxyphenole, wie beispielsweise Brenzcatechin (1,2-Dihydroxybenzol) oder Benzochinon; Aminophenole, wie z.B. p-Aminophenol; Nitrosophenole, wie z.B. p-Nitrosophenol; Alkoxyphenole, wie beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Di-tert.-Butyl-4-methoxyphenol; Tocipherole, wie z.B. α-Tocopherol sowie 2,3-Dihydro-2,2-dimethyl-7-hydroxybenzofuran (2,2-Dimethyl-7-hydroxycumaran), aromatische Amine, wie z.B. N,N-Diphenylamin oder N-Nitrosodiphenylamin; Phenylendiamine, wie z.B. N,N'-Dialkyl-p-phenylendiamin, wobei die Alkylreste gleich oder verschieden sein können und jeweils unabhängig voneinander aus 1 bis 4 Kohlenstoffatomen bestehen und geradkettig oder verzweigt sein können, wie z.B. N,N'-Dimethyl-p-phenylendiamin oder N,N'-Diethyl-p-phenylendiamin, Hydroxylamine, wie z.B. N,N-Diethylhydroxylamin, Imine, wie z.B. Methylethylimin oder Methylen violett, Sulfonamide, wie z.B. N-Methyl-4-toluolsulfonamid oder N-tert.-Butyl-4-toluolsulfonamid, Oxime, wie Aldoxime, Ketoxime oder Amidoxime, wie z.B. Diethylketoxim, Methylethylketoxim oder Salicyladoxim, phosphorhaltige Verbindungen, wie z.B. Triphenylphosphin, Triphenylphosphit, Triethylphosphit, hypophsophorige Säure oder Alkylester der Phosphorigen Säuren; schwefelhaltige Verbindungen wie z.B. Diphenylsulfid oder Phenothiazin; Metallsalze, wie Kupfer- oder Mangan-, Cer-, Nickel-, Chromsalze, beispielsweise -chloride, -sulfate, -salicylate, -tosylate, acrylate oder -acetate, wie z.B. Kupferacetat, Kupfer(II)chlorid, Kupfersalicylat, Cer(III)acetat oder Cer(III)ethythexanoat, oder Gemische davon sein.

Bevorzugt sind Hydrochinon, Hydrochinonmonomethylether, Phenothiazin, 4-Hydroxy-2,2,6,6-tetramethylpiperidin-N-oxyl, 4-Oxo-2,2,6,6-tetramethylpiperidin-N-oxyl, 2-tert.-Butylphenol, 4-tert.-Butylphenol, 2,4-Di-tert.-butylphenol, 2-tert.-Butyl-4-methylphenol, 6-tert.-Butyl-2,4-dimethylphenol, 2,6-Di-tert.-butyl-4-methylphenol und 2-Methyl-4-tert.-butylphenol

Besonders bevorzugt ist Hydrochinonmonomethylether (MEHQ).

Vorteilhaft kann zusätzlich als Polymerisationsinhibitor Sauerstoff eingesetzt werden.

Zur weiteren Stabilisierung kann ein sauerstoffhaltiges Gas, bevorzugt Luft oder ein Gemisch aus Luft und Stickstoff (Magerluft), anwesend sein.

Die Umesterungsreaktion (Schritte (i) und (ii)) wird im Allgemeinen bei einer Temperatur von 60 bis 140 °C, bevorzugt 70 bis 110 °C durchgeführt. Dabei wird ein Azeotrop aus Schleppmittel und Alkohol kontinuierlich abdestilliert.

Geeignete, mit Methanol oder Ethanol ein azeotrop siedendes Gemisch bildende Schleppmittel sind zunächst Methylacrylat und Methylmethacrylat sowie Ethylacrylat und Ethyl(meth)acrylat selbst. Als separate Schleppmittel geeignet sind unter anderem Cyclohexan, Methylcyclohexan, Benzol, Toluol, Hexane und Heptane und Mischungen hieraus. Bevorzugt sind Methylacrylat, Methylmethacrylat, Ethylacrylat und Ethyl(meth)acrylat sowie Mischungen von diesen mit n-Heptan und Cyclohexan. Der Begriff Schleppmittel umfasst in diesem Sinne das Edukt selbst sowie gegebenenfalls ein zusätzlich eingesetztes separates Lösungsmittel.

In einer bevorzugten Ausführungsform wird kein separates Lösungsmittel als Schleppmittel eingesetzt. In diesem Fall fungiert das Edukt Alkyl(meth)acrylat selbst als Schleppmittel.

Das Schleppmittel kann anschließend wieder im Reaktor ergänzt werden. Hierzu wird das azeotrope Gemisch aus Alkohol und Schleppmittel in einer bevorzugten Ausführungsform über eine geeignete Kolonne abdestilliert, in einem Mischgefäß mit Wasser gerührt und dann in einen Phasenseparator überführt, wobei sich der Alkohol, im Allgemeinen Methanol oder Ethanol, in Wasser löst und sich die organische Phase als obere Schicht abscheidet. Die organische Phase wird vorzugsweise über den Kopf der Kolonne dem Reaktionsgemisch wieder zugeführt und damit bis auf geringe Verluste im Kreislauf geführt. Es kann aber auch alternativ frisches Schleppmittel hinzugeführt werden und eine Aufarbeitung des Schleppmittel-Alkohol-Gemisches in einem separaten Schritt erfolgen oder auf die Ergänzung des Schleppmittels ganz oder teilweise verzichtet werden.

Im Allgemeinen wird Alkyl(meth)acrylat in stöchiometrischem Überschuss eingesetzt. Vorzugsweise beträgt der Überschuss an Methyl(meth)acrylat pro zu veresternder Hydroxylgruppe 5 bis 200 mol-%, besonders bevorzugt 5 bis 100 mol-%, insbesondere 5 bis 50 mol-%.

Der Katalysator wird in einer Konzentration von 0,1-10 mol-% bezogen auf die Menge an Heonon eingesetzt, bevorzugt in einer Konzentration von 0,1 bis 5 mol-%.

Die Umesterung kann bei Atmosphärendruck, aber auch bei Überdruck oder Unterdruck durchgeführt werden. Im Allgemeinen wird sie bei 300 bis 1000 mbar, bevorzugt bei 800 -1000 mbar (Atmosphärendruck = 1000 mbar) durchgeführt. Die Reaktionszeit beträgt im Allgemeinen 1 h bis 24 Stunden, vorzugsweise 3 bis 18 Stunden, besonders bevorzugt 6 bis 12 h. Die Umesterung (Schritte (i) und (ii)) kann kontinuierlich, beispielsweise in einer Rührkesselkaskade oder diskontinuierlich erfolgen.

Die Reaktion kann in allen für eine solche Umsetzung geeigneten Reaktoren durchgeführt werden. Solche Reaktoren sind dem Fachmann bekannt. Bevorzugt erfolgt die Umsetzung in einem Rührkesselreaktor.

Zur Durchmischung des Ansatzes können beliebige Verfahren eingesetzt werden wie z.B. Rührvorrichtungen. Die Durchmischung kann auch durch Einspeisen eines Gases, vorzugsweise eines Sauerstoff-haltigen Gases erfolgen.

Das Entfernen des gebildeten Alkohols, im Allgemeinen Methanol oder Ethanol, erfolgt kontinuierlich oder schrittweise in an sich bekannter Weise durch azeotrope Destillation in Gegenwart eines Schleppmittels. Zusätzlich kann Methanol auch durch Strippen mit einem Gas entfernt werden.

In einer bevorzugten Ausführungsform wird aus dem in Schritt (ii) abdestillierten Azeotrop aus Schleppmittel und Alkohol der Alkohol durch Waschen mit Wasser abgetrennt und das Schleppmittel in den Reaktionsbehälter zurückgeführt.

Die Schritte (i) und (ii) werden durchgeführt, bis das eingesetzte Heonon im Wesentlichen vollständig umgesetzt ist. Dies ist der Fall, wenn Heonon zu 95 %, bevorzugt zu 98 %, besonders bevorzugt zu 99 % umgesetzt ist.

Anschließend werden die Schritte (iii) und (iv) durchgeführt, die auch in umgekehrter Reihenfolge durchgeführt werden können.

In Schritt (iii) wird zu dem das Heonon(meth)acrylat enthaltenden Produktgemisch Wasser zugegeben, wodurch der Titan(IV) oder Zirkonium(IV) enthaltende Katalysator zu dem entsprechenden Hydroxid hydrolysiert wird. Das schwerlösliche Hydrolysat wird anschließend abgetrennt, z.B. durch Filtration oder Zentrifugation.

Die Filtration kann beispielsweise mit einer Druckfilternutsche durchgeführt werden. Verfahrenstechnisch können für eine Filtration im erfindungsgemäßen Verfahren alle an sich bekannten Filtrationsverfahren und -apparate eingesetzt werden, z.B. solche, die in Ullmann's Encyclopedia of Industrial Chemistry, 7th ed, 2013 Electronic Release, Kapitel: Filtration, 1. Fundamentals und Filtration 2. Equipment, beschrieben sind. Beispielsweise können dies Kerzenfilter, Filterpressen, Tellerdruckfilter, Beutelfilter oder Trommelfilter sein. Vorzugsweise werden Kerzenfilter oder Tellerdruckfilter eingesetzt. Die Filtration kann mit oder ohne Filterhilfsmittel durchgeführt werden. Geeignete Filterhilfsmittel sind Filterhilfsmittel basierend auf Kieselgur, Perlit und Cellulose.

Geeignete Zentrifugen und auch Separatoren sind dem Experten bekannt. Verfahrenstechnisch können für eine Zentrifugation im erfindungsgemäßen Verfahren alle an sich bekannten Zentrifugationsverfahren und -apparate eingesetzt werden, z.B. solche, die in Ullmann's Encyclopedia of Industrial Chemistry, 7th ed, 2013 Electronic Release, Kapitel: Centrifuges, Filtering und Centrifuges, Sedimenting, beschrieben sind.

In einer bevorzugten Ausführungsform werden dann anschließend in den Destillationsschritten (iv) und (v) nicht umgesetztes Alkyl(meth)acrylat sowie Wasser aus dem Produktgemisch abdestilliert. Diese Destillation erfolgt im Allgemeinen bei einer Temperatur von 40 bis 100 °C, bevorzugt 60 bis 80 °C und einem variablen Druck von 10 bis 700 mbar. Zusätzlich können diese Komponenten auch durch Strippen mit einem Gas, vorzugsweise einem sauerstoffhaltigen Gas entfernt werden.

Wird kein separates Schleppmittel eingesetzt, so werden die Schritte (iv) und (v) bevorzugt in einem gemeinsamen Destillationsschritt durchgeführt. Wird ein separates Schleppmittel eingesetzt, so wird Schritt (iv) bevorzugt vor Schritt (iii) durchgeführt.

Die destillative Abtrennung erfolgt beispielsweise in einem Rührkessel mit Doppelwandheizung und/oder innenliegenden Heizschlangen unter vermindertem Druck.

Selbstverständlich kann die Destillation auch in einem Fallfilm- oder Dünnschichtverdampfer erfolgen. Dazu wird das Reaktionsgemisch, bevorzugt mehrmals im Kreislauf, unter vermindertem Druck, beispielsweise bei 20 bis 700 mbar, bevorzugt 30 bis 500, besonders bevorzugt 50 bis 150 mbar und einer Temperatur von 40 bis 80 °C durch den Apparat geführt.

Vorteilhaft kann ein inertes Gas, bevorzugt ein sauerstoffhaltiges Gas, besonders bevorzugt Luft oder ein Gemisch aus Luft und Stickstoff (Magerluft) in den Destillationsapparat eingeleitet werden, beispielsweise 0,1 bis 1, bevorzugt 0,2 bis 0,8 und besonders bevorzugt 0,3 bis 0,7 m³/m³h, bezogen auf das Volumen des Reaktionsgemisches.

Nach Durchführung der Schritte (iii), (iv) und (v) verbleibt ein Produkt als Sumpfprodukt, welches die oben beschriebene Reinheit aufweist.

Die Erfindung wird durch die nachstehenden Beispiele näher ausgeführt.

### Beispiele

### Beispiel 1

### Heononacrylat durch Umesterung mit Titan-haltigem Katalysator

In einem 4L-Planschliffreaktor mit aufgesetzter Kolonne (Montz A3-500 Packung), Kühler, Flüssigkeitsteiler, Ankerrührer sowie Magerlufteinleitung werden Ethylacrylat (1500 g), MeHQ (2 g), Acrylsäure (2,5 g) sowie Heonon (694 g) vorgelegt und unter Magerlufteinleitung und Rühren hochgeheizt. Es wird zur Entwässerung Ethylacrylat abdestilliert und frisches Ethylacrylat ergänzt. Titantetraisopropoxylat (14 g) wird bei einer Sumpftemperatur von 79°C zudosiert und weiter auf 102°C Sumpftemperatur aufgeheizt. Nach Siedebeginn wird ein Rücklaufverhältnis von 5:2 eingestellt. Ethylacrylat wird portionsweise zudosiert, in den Mengen, die dem Destillat entsprechen. Nach 5,5 h werden 20 g Katalysator nachdosiert. Die Sumpftemperatur steigt im Verlauf der Reaktion auf 104°C an. In regelmäßigen Abständen werden Sumpf- und Destillatproben gezogen, um den Verlauf der Reaktion zu beobachten. Nach 17 h Reaktionszeit zeigt das GC (FI.-%) einen Gehalt von 98,8% Heononacrylat sowie 1,2% Restalkohol an (Ethylacrylat herausgerechnet). Es werden 150 ml Wasser hinzugegeben, die Reaktionsmischung über eine mit Sand gefüllte Fritte filtriert und im Vakuum eingeengt.

Das Produkt wird nach einer Klärfiltration in 830 g Ausbeute mit einer Reinheit von 96% (GC-FI-%) erhalten. Im GC sind zwei unbekannte Nebenprodukte zu erkennen mit insgesamt 1,7%, aber kein Michael-Addukt. Der Restalkoholgehalt beträgt 2,2%.

### Beispiel 2

### Heononacrylat durch Umesterung mit Titan-haltigem Katalysator

In einem 4L-Planschliffreaktor mit aufgesetzter Kolonne (Montz A3-500 Packung), Kühler, Flüssigkeitsteiler, Ankerrührer sowie Magerlufteinleitung werden Ethylacrylat (1500 g), MeHQ (2 g), Phosphorsäure 85% (12 g) sowie Heonon (694 g) vorgelegt und unter Magerlufteinleitung und Rühren hochgeheizt. Es wird zur Entwässerung Ethylacrylat abdestilliert und frisches Ethylacrylat ergänzt. Titantetraisopropoxylat (14 g) wird bei einer Sumpftemperatur von 76°C zudosiert und weiter auf 103°C Sumpftemperatur aufgeheizt. Nach Siedebeginn wird ein Rücklaufverhältnis von 5:2 eingestellt. Nach 4 h werden 14 g Katalysator nachdosiert. Die Sumpftemperatur steigt im Verlauf der Reaktion auf 104°C an. In regelmäßigen Abständen werden Sumpf- und Destillatproben gezogen, um den Verlauf der Reaktion zu beobachten. Nach 16,5 h Reaktionszeit zeigt das GC (FI.-%) einen Gehalt von 97% Heononacrylat sowie 0,8% Restalkohol an (Ethylacrylat herausgerechnet). Es werden 150 ml Wasser hinzugegeben, die Reaktionsmischung über eine mit Sand gefüllte Fritte und einen Seitz-Filter filtriert.

Die Reaktionsmischung wird im Vakuum konzentriert. Das Produkt wird in 787 g Ausbeute mit einer Reinheit von 95,4% (GC-FI.-%) erhalten. Im GC ist ein unbekanntes Nebenprodukt zu erkennen mit insgesamt 1,1%, aber kein Michael-Addukt. Der Restalkoholgehalt beträgt 1,6%, der Ethylacrylat-Gehalt 1,7%.

### Vergleichsbeispiel 1

### Heononacrylat durch Umesterung mit Titan-haltigem Katalysator

In einem 0,75L Planschliffreaktor mit aufgesetzter Kolonne, Kühler, Flüssigkeitsteiler, Ankerrührer sowie Magerlufteinleitung werden Ethylacrylat (500 g), MeHQ (0,23 g), PTZ (0,02g) sowie Heonon (150 g) vorgelegt und unter Magerlufteinleitung und Rühren mit einer Badtemperatur von 80°C hochgeheizt. Es wird zur Entwässerung Ethylacrylat abdestilliert und frisches Ethylacrylat ergänzt. Titantetraisopropoxylat (5 g) wird zudosiert und weiter auf 97°C Sumpftemperatur aufgeheizt. Es wird ein Vakuum von 900 mbar angelegt, das im Verlauf der Reaktion auf bis zu 970 mbar angehoben wird. Nach Siedebeginn wird ein Rücklaufverhältnis von 10:1 eingestellt. Die Sumpftemperatur steigt im Verlauf der Reaktion auf 105°C an. In regelmäßigen Abständen werden Sumpf- und Destillatproben gezogen, um den Verlauf der Reaktion zu beobachten. Nach 5 h Reaktionszeit zeigt das GC (FI.-%) einen Gehalt von 43% Heononacrylat, 45% Michael-Addukt (Michael-Addukt des Alkohols an Ethylacrylat identifiziert via GC-MS) sowie 12% Restalkohol an (Ethylacrylat herausgerechnet). Der Versuch wird abgebrochen.

### Vergleichsbeispiel 2

### Heononacrylat durch Umesterung mit Zirkon-haltigem Katalysator

In einem 4L-Planschliffreaktor mit aufgesetzter Kolonne (Montz A3-500 Packung), Kühler, Flüssigkeitsteiler, Ankerrührer sowie Magerlufteinleitung werden Ethylacrylat (1500 g), MeHQ (1,97 g), sowie Heonon (694 g) vorgelegt und unter Magerlufteinleitung und Rühren auf eine Sumpftemperatur von 45°C hochgeheizt. Zr(IV)-Acetylacetonat (12,2 g) wird zudosiert und weiter auf eine Sumpftemperatur von 76-80°C aufgeheizt bei einem Druck von 930 mbar. Das Rücklaufverhältnis wird variiert von 10:1 bis 5:2. In regelmäßigen Abständen werden Sumpf- und Destillatproben gezogen, um den Verlauf der Reaktion zu beobachten. Nach 11 h Reaktionszeit zeigt das GC (FI.-%) einen Gehalt von 15% Heononacrylat, 20% Michael-Addukt (Michael-Addukt des Alkohols an Ethylacrylat identifiziert via GC-MS), in Summe >3% unbekannte Nebenprodukte sowie 60% Restalkohol an (Ethylacrylat herausgerechnet). Der Versuch wird abgebrochen. Bei dem Nebenprodukt handelt es sich um das Michael-Addukt des Alkohols an Ethylacrylat.

## Patentansprüche

1. Verfahren zur Herstellung von Heonon(meth)acrylat durch Umesterung von Alkyl(meth)acrylat mit 2-Hydroxyethyloxazolidinon auch genannt Heonon, umfassend die Schritte:
(i) Reagieren lassen von Alkyl(meth)acrylat mit Heonon in Gegenwart eines Titan(IV) oder Zirkonium(IV) enthaltenden Katalysators und eines Stabilisators in Gegenwart eines Schleppmittels, das mit dem in dem Alkyl(meth)acrylat gebundenen Alkohol ein Azeotrop bildet,
(ii) kontinuierliches Abdestillieren des Azeotrops aus Schleppmittel und Alkohol, wobei die Schritte (i) und (ii) gleichzeitig durchgeführt werden, bis Heonon im Wesentlichen vollständig umgesetzt ist,
(iii) Zugabe von Wasser zu dem in den Schritten (i) und (ii) erhaltenen, Heonon(meth)acrylat enthaltenden Produktgemisch und Abtrennung des Hydrolysats des Titan(IV) oder Zirkonium(IV) enthaltenden Katalysators durch Filtration,
(iv) Abdestillieren von nicht umgesetztem Alkyl(meth)acrylat und Schleppmittel aus dem Produktgemisch,
(v) Abdestillieren von Wasser aus dem Produktgemisch,
wobei Schritt (iv) auch vor Schritt (iii) durchgeführt werden kann und die Schritte (iv) und (v) auch in einem Destillationsschritt durchgeführt werden können,
**dadurch gekennzeichnet, dass** die Schritte (i) und (ii) in Gegenwart einer zusätzlichen anorganischen oder organischen Säure durchgeführt werden.

2. Verfahren nach Anspruch 1, **dadurch gekennzeichnet, dass** das Schleppmittel das Alkyl(meth)acrylat ist.

3. Verfahren nach Anspruch 1, **dadurch gekennzeichnet, dass** das Schleppmittel ein separates, von Alkyl(meth)acrylat verschiedenes Lösungsmittel ist.

4. Verfahren nach Anspruch 3, **dadurch gekennzeichnet, dass** das Schleppmittel ausgewählt ist aus der Gruppe bestehend aus n-Heptan und Cyclohexan.

5. Verfahren nach einem der Ansprüche 1 bis 4, **dadurch gekennzeichnet, dass** die Schritte (iv) und (v) in einem gemeinsamen Destillationsschritt durchgeführt werden.

6. Verfahren nach einem der Ansprüche 1 bis 5, **dadurch gekennzeichnet, dass** das Alkyl(meth)acrylat das Methyl- oder Ethyl(meth)acrylat ist.

7. Verfahren nach einem der Ansprüche 1 bis 6, **dadurch gekennzeichnet, dass** der Katalysator Titan(IV)tetraisopropylat enthält.

8. Verfahren nach einem der Ansprüche 1 bis 7, **dadurch gekennzeichnet, dass** der Stabilisator Methylhydrochinon ist.

9. Verfahren nach einem der Ansprüche 1 bis 8, **dadurch gekennzeichnet, dass** aus dem in Schritt (ii) abdestillierten Azeotrop aus Schleppmittel und Alkohol durch Waschen mit Wasser der Alkohol abgetrennt und das Schleppmittel in den Reaktionsbehälter zurückgeführt wird.

10. Verfahren nach einem der Ansprüche 1 bis 9, **dadurch gekennzeichnet, dass** nach Schritt (v) ein Heonon(meth)acrylat mit einem Nebenproduktgehalt von < 2 Gew.-% erhalten wird.

## Claims

1. A process for preparing heonone (meth)acrylate by transesterification of alkyl (meth)acrylate with 2-hydroxyethyloxazolidinone, also called heonone, which comprises the steps:
(i) reaction of alkyl (meth)acrylate with heonone in the presence of a catalyst comprising titanium(IV) or zirconium(IV) and a stabilizer in the presence of an entrainer which forms an azeotrope with the alcohol bound in the alkyl (meth)acrylate,
(ii) continuous removal by distillation of the azeotrope of entrainer and alcohol, with steps (i) and (ii) being carried out simultaneously until heonone has been essentially completely reacted,
(iii) addition of water to the product mixture comprising heonone (meth)acrylate obtained in steps (i) and (ii) and removal of the hydrolyzate of the catalyst comprising titanium(IV) or zirconium(IV) by filtration,
(iv) distillation of unreacted alkyl (meth)acrylate and entrainer from the product mixture,
(v) distillation of water from the product mixture, with step (iv) also being able to be carried out before step (iii) and steps (iv) and (v) also being able to be carried out in a distillation step, wherein steps (i) and (ii) are carried out in the presence of an additional inorganic or organic acid.

2. The process according to claim 1, wherein the entrainer is the alkyl (meth)acrylate.

3. The process according to claim 1, wherein the entrainer is a separate solvent different from alkyl (meth)acrylate.

4. The process according to claim 3, wherein the entrainer is selected from the group consisting of n-heptane and cyclohexane.

5. The process according to any of claims 1 to 4, wherein steps (iv) and (v) are carried out in a joint distillation step.

6. The process according to any of claims 1 to 5, wherein the alkyl (meth)acrylate is methyl or ethyl (meth)acrylate.

7. The process according to any of claims 1 to 6, wherein the catalyst comprises titanium(IV) tetraisopropoxide.

8. The process according to any of claims 1 to 7, wherein the stabilizer is methylhydroquinone.

9. The process according to any of claims 1 to 8, wherein the alcohol is separated off from the azeotrope of entrainer and alcohol distilled off in step (ii) by scrubbing with water and the entrainer is recirculated to the reaction vessel.

10. The process according to any of claims 1 to 9, wherein a heonone (meth)acrylate having a byproduct content of < 2% by weight is obtained after step (v).

## Revendications

1. Procédé de fabrication de (méth)acrylate d'héonone par transestérification de (méth)acrylate d'alkyle avec de la 2-hydroxyéthyloxazolidinone, également nommée héonone, comprenant les étapes suivantes :
(i) la mise en réaction d'un (méth)acrylate d'alkyle avec de l'héonone en présence d'un catalyseur contenant du titane (IV) ou du zirconium (IV) et d'un stabilisateur en présence d'un agent d'entraînement, qui forme un azéotrope avec l'alcool relié dans le (méth)acrylate d'alkyle,
(ii) la distillation continue de l'azéotrope constitué par l'agent d'entraînement et l'alcool, les étapes (i) et (ii) étant réalisées simultanément, jusqu'à ce que l'héonone ait été transformée essentiellement en totalité,
(iii) l'ajout d'eau au mélange de produits contenant du (méth)acrylate d'héonone obtenu aux étapes (i) et (ii) et la séparation du produit hydrolysé du catalyseur contenant du titane (IV) ou du zirconium (IV) par filtration,
(iv) la distillation du (méth)acrylate d'alkyle non réagi et de l'agent d'entraînement du mélange de produits,
(v) la distillation de l'eau du mélange de produits, l'étape (iv) pouvant également être réalisée avant l'étape (iii) et les étapes (iv) et (v) pouvant également être réalisées en une étape de distillation, **caractérisé en ce que** les étapes (i) et (ii) sont réalisées en présence d'un acide inorganique ou organique supplémentaire.

2. Procédé selon la revendication 1, **caractérisé en ce que** l'agent d'entraînement est le (méth)acrylate d'alkyle.

3. Procédé selon la revendication 1, **caractérisé en ce que** l'agent d'entraînement est un solvant séparé, différent du (méth)acrylate d'alkyle.

4. Procédé selon la revendication 3, **caractérisé en ce que** l'agent d'entraînement est choisi dans le groupe constitué par le n-heptane et le cyclohexane.

5. Procédé selon l'une quelconque des revendications 1 à 4, **caractérisé en ce que** les étapes (iv) et (v) sont réalisées en une étape de distillation commune.

6. Procédé selon l'une quelconque des revendications 1 à 5, **caractérisé en ce que** le (méth)acrylate d'alkyle est le (méth)acrylate de méthyle ou d'éthyle.

7. Procédé selon l'une quelconque des revendications 1 à 6, **caractérisé en ce que** le catalyseur contient du tétraisopropylate de titane (IV).

8. Procédé selon l'une quelconque des revendications 1 à 7, **caractérisé en ce que** le stabilisateur est la méthylhydroquinone.

9. Procédé selon l'une quelconque des revendications 1 à 8, **caractérisé en ce que** l'alcool est séparé de l'azéotrope constitué par l'agent d'entraînement et l'alcool distillé à l'étape (ii) par lavage avec de l'eau, et l'agent d'entraînement est recyclé dans le contenant de réaction.

10. Procédé selon l'une quelconque des revendications 1 à 9, **caractérisé en ce qu'**un (méth)acrylate d'héonone ayant une teneur en produits secondaires < 2 % en poids est obtenu à l'étape (v).
